# EUROPEAN PATENT SPECIFICATION

(11) **EP 1 423 077 B1**
(45) Date of publication and mention of the grant of the patent: **31.03.2010**
(21) Application number: 02759039.7
(22) Date of filing: 21.08.2002
(51) Int. Cl.: A61F 13/64

(54) **BELTED ABSORBENT ARTICLE AND A METHOD OF PRODUCING A LAMINATE FOR USE AS BELT MATERIAL**
SAUGFÄHIGER ARTIKEL MIT GÜRTEL UND VERFAHREN ZUR HERSTELLUNG EINES VERBUNDMATERIALS ZUR VERWENDUNG ALS GÜRTELMATERIAL
ARTICLE ABSORBANT A CEINTURE ET PROCEDE DE PRODUCTION D'UN LAMINE UTILISE COMME MATERIAU DE CEINTURE

(30) Priority: 22.08.2001 SE 0102800
(43) Date of publication of application: 02.06.2004
(73) Proprietor: SCA Hygiene Products AB, 405 03 Göteborg (SE)
(72) Inventor: ALMBERG, Christian, S-435 41 Mölnlycke (SE); KUSIBOJOSKA, Liljana, S-254 39 Helsingborg (SE)
(74) Representative: Egeröd, Lisbeth
(86) International application number: PCT/SE2002/001496
(87) International publication number: WO 2003/017904

(56) References cited:
- EP-A- 1 104 692
- WO-A-95/05793
- US-A- 5 628 741
- US-A- 5 807 368

## Description

### Technical field

The present invention refers to an absorbent article such as a diaper and an incontinence guard comprising a liquid permeable topsheet, a liquid impermeable backsheet and an absorbent body enclosed therebetween, said article having a front portion, a rear portion and a crotch portion therebetween, and further is provided with a pair of belt members attached to the rear portion , alternatively to the front portion, of the article and which are intended to be fastened together around the waist of the wearer and where said front portion, alternatively said rear portion, is provided with fastening means intended to be fastened to the belt members, in such a way that the article will assume a pantlike shape, where the belt members form a part of the waist portions of the pant. The invention further refers to a method of producing a flexible laminate for use as belt members on an absorbent article such as a diaper and an incontinence guard

### Background of the invention

Diapers and incontinence guards for incontinent adults usually have a garment portion holding an absorbent body in place against the user's body and attachment means which hold the garment portion in place also when the user is moving. A common type of attachment means are adhesive tapes or hook and loop fasteners of the touch-and-close type which directly attach the front and rear portions of the absorbent article to each other. It is further known, through e g EP-A-0 287 388, EP-A-0 409 307, EP-A-0 605 012 and FR-A-2 586 558, to attach the front and rear portions of the article by means of a belt. The belt members are usually attached to the rear portion of the diaper and are intended to be fastened together around the waist of the wearer and fastening means provided at the front portion of the diaper are then intended to be fastened to the outside of the belt members. The belt provides improved possibilities to adjust the fit of the diaper. The belt further provides a simplified change of diaper or incontinence guard, especially when the wearer is standing.

One problem with these belts is that they may cause skin irritations to the user, due to that the belt is in direct contact with the skin of the wearer and has to be tightened relatively strongly in order to have a satisfactory fit and security against leakage of the diaper or incontinence guard. By the tight contact and friction between the belt and the skin there will be a mechanic wear of the skin which gives rise to irritation and even skin injuries. It is therefor important that the material used to form the inside of the belt is soft and skin-friendly. Belt materials dealing with this problem are disclosed in WO 00/27330 and in WO 01/00129.

As mentioned above the outside of the belt should serve as a receiving surface for the fastening means provided on one of the belt members and on the front portion of the diaper or incontinence guard. For a hook-and-loop type fastening means the outside of the belt should serve as a loop material cooperating with a hook material constituting said fastening means. A nonwoven material is from cost point of view preferred to use as a loop material, but a substantially plane and smooth nonwoven material does not always provide the necessary shear and peel strength required to withstand the forces applied thereto during normal use of the article. A shear force is applied in a plane substantially parallel to the connected surfaces of the hook and loop elements, while a peel force is applied in a direction substantially perpendicular to the connected surfaces of the hook and loop elements.

WO 97/19665 discloses a loop fastening material in the form of a creped nonwoven layer attached to a support layer by a bonding pattern provided by heat or ultrasonic. The creping of the material is told to improve the loop function of the material. There is no disclosure of this material being used as a belt material.

### Object and most important features of the invention

An object of the present invention is to provide a belt for absorbent articles which is comfortable to wear, is resistant to tearing and which has an improved loop function. This has according to the invention been provided by the fact that the belt members comprise a flexible laminate of at least three layers, a first outer layer, a middle layer and a second outer layer of fibrous material bonded together in a bonding pattern provided by ultrasonic, laser and/or heat, said bonding pattern having a bonding area of no more than 10% , said first outer layer and said middle layer of the laminate having a creped structure of a plurality of raised areas separated by a plurality of non-raised area formed by the bonding sites of said bonding pattern, wherein the creped structure of the first outer layer is more distinct with a greater height of said raised areas as compared to the middle layer.

According to one embodiment the second outer layer of said laminate is substantially smooth and uncreped.

The second outer layer of the laminate is preferably used as the internal side of the belt members intended to be facing the wearer, while the first outer layer of the laminate is used as the external side of the belt members intended to act as receiving surface for said fastening means, and is especially used as a loop material for a complementary hook material of a hook-and-loop type fastening means.

According to further preferred embodiments the bonding pattern has a bonding area of no more than 8% and preferably no more than 5%

In a further aspect of the invention the bonding pattern has a tightness of bonding sites of between 1 and 15 bonding sites per cm² and preferably between 1 and 10 bonding sites per cm².

According to one embodiment said middle layer is a relatively tear strong fibrous material comprising continuous filaments, such as a spunbond and/or meltblown material.

The invention further refers to a method of producing a flexible laminate for use as belt material on an absorbent article such as a diaper and an incontinence guard, said method comprising the steps of: binding together at least three layers, a first outer layer, a middle layer and a second outer layer of fibrous material in a bonding pattern provided by ultrasonic, laser and/or heat, said bonding pattern having a bonding area of no more than 10% , said layers of the laminate exhibiting different web tensions and/or web speeds during bonding, so that said first outer layer exhibits the lowest web tension and/or lowest web speed, the second outer layer exhibits the highest web tension and/or highest web speed and the middle layer exhibits a web tension and/or web speed that is higher than that of the first outer layer and lower than that of the second outer layer.

In one embodiment the second outer layer has a web tension and/or web speed during bonding that is between 15 and 50% and preferably between 18 and 33% higher than that of the first outer layer. According to a further embodiment the middle layer has a web tension and/or web speed during bonding that is between 5 and 40% and preferably between 9 and 18% higher than that of the first outer layer.

### Description of drawings

The invention will in the following be closer described witch reference to an embodiment shown in the accompanying drawings.
Fig. 1 shows a schematic perspective view of a belt diaper according to the invention.
Fig. 2 shows schematically a cross section through a laminate according to the invention.
Fig. 3 is a schematic side view of a device for performing the method according to the invention.

### Description of embodiments

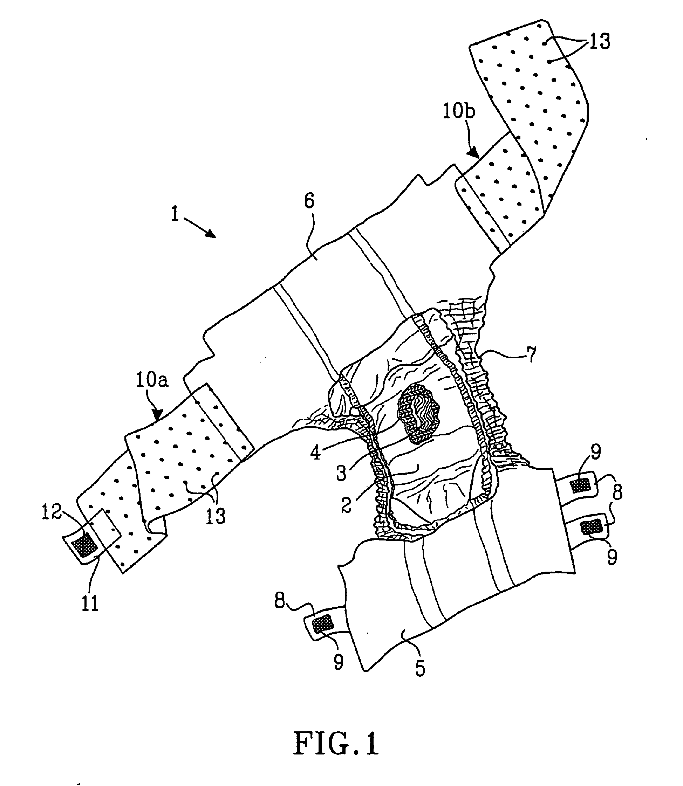

Fig. 1 of the drawings shows an embodiment of a diaper or incontinence guard 1 comprising a liquid permeable topsheet 2, a liquid impermeable backsheet 3 and an absorbent body 4 enclosed therebetween. The liquid permeable topsheet 2 can be any material used for this purpose, for example a nonwoven material, such as a spunbond material of continuous filaments, a meltblown material, a thermobonded fibrous web such as a carded fibrous web. The topsheet may also be a layer of so called tow fibers bonded in a bonding pattern or a perforated plastic film.

The liquid impermeable backsheet 3 may also be any material used for this purpose, such as a plastic film, a nonwoven material coated with a liquid impervious material, a hydrophobic nonwoven material which resists liquid penetration and/or a laminate of plastic film and nonwoven material. Breathable materials which are permeable to air and water vapour but which resist liquid penetration at least up to a certain pressure may also be used as backsheet materials.

The topsheet 2 and the backsheet material 3 have a somewhat greater extension in the plane than the absorbent body 4 and extends outside the edges thereof. The layers 2 and 3 are connected to each other within the projecting portions thereof, e g by gluing or welding by heat or ultrasonic.

The absorbent body 4 can be of any kind used for this purpose. Examples of commonly used absorbent materials are cellulosic fluff pulp, tissue layers, highly absorbent polymers (so called superabsorbents), absorbent foam materials, absorbent nonwoven materials or the like. It is common to combine cellulosic fluff pulp with superabsorbents in an absorbent body. It is also common to have absorbent bodies comprising layers of different material with different properties with respect to liquid acquisition capacity, liquid distribution capacity and storage capacity. It is well-known to the person skilled in the art and does therefore not have to be described in detail. The thin absorbent bodies which are common in for example baby diapers and incontinence guards often comprise a compressed mixed or layered structure of cellulosic fluff pulp and superabsorbent.

The diaper is intended to enclose the lower part of the wearer's trunk like a pair of absorbent pants. It comprises a front portion 5 intended during use to be worn on the front part of the user's body, a rear portion 6 intended during use to be worn on the rear part of the user's body, and a more narrow crotch portion 7 located between the front and rear portions and which is intended to be worn in the crotch part of the user between the legs. The front portion 5 is provided with a pair of tabs 8 carrying attachment means such as a hook material 9 of a so called hook-and-loop type fastener or other type of attachment means such as adhesive tape.

The term "hook material" is used to designate the portion of a mechanical fastening means having engaging "hook" element. However it is not intended to limit the shape of the engaging elements to include only "hooks" but encompasses any shape of engaging elements, unidirectional or bidirectional, known in the art to mechanically engage a complementary loop fastening material.

A pair of belt members 10a and b are with one end attached, e g glued or ultrasonically welded to the rear part 6 of the diaper. The belt members 10 a, b are with their opposite ends intended to be fastened together, e g by a tab 11 on one belt member 10a, said tab carrying a hook material 12 of a so called hook-and-loop type fastener. The hook material 12 on the tab 11 is intended to attach to the outside of the opposite belt member 10 b. Instead of hook-and-loop type fastener 12 there may be another type of optional attachment means, such as adhesive tape.

The tabs 8 carrying a hook material or corresponding attachment means of the front portion 5 are intended to be attached to the outside of the belt members 10 a, b in order to fasten together the diaper to the desired pantlike shape.

In an alternative embodiment the belt members 10 a, b are attached to the front portion 5 of the diaper and are then fastened together on the back of the wearer. Tabs 8 carrying fastening means, for example a hook material or an adhesive tape, are in this case provided at the rear portion 6 of the diaper.

The outside of the belt members 10 a, b should act as a reception surface cooperating with the fastening means on tabs 8 and 11. For hook-and-loop fasteners the material on the outside of the belt portions should serve as a loop material. The term "loop" in this respect is not limited only to materials in which discrete, separately formed loops of material are adapted to receive and engage the hook elements of a complementary hook material, but the loop material also includes fibrous nonwoven in which the individual fibers function to engage the hook elements without such fibers being formed into discrete loops.

For tape fasteners the material on the outside of the belt members 10 a, b should serve as attachment surface for adhesive tapes. Certain nonwoven materials will function both as loop material for hook-and-loop fasteners and as attachments surface admitting refastening of an adhesive tape. This is disclosed in WO 01/00129.

The width of the belt members should be between 5 and 20 cm, preferably between 7 and 15 cm.

The belt members according to this invention comprise a flexible laminate of at least three fibrous layers 14, 15 and 16, first and second outer layers 14, 15 and a middle layer 16, of fibrous material bonded together in a bonding pattern 13 provided by ultrasonic, laser and/or heat. At least some of the fibers in the layers of fibrous material should therefore be meltable by such bonding techniques. The bonding pattern 13 should have a bonding area of no more than 10% . The fibrous materials are preferably nonwoven materials, such as spunbond, meltblown, carded bonded webs, thermobonded webs etc.

The first outer layer 14 and the middle layer 16 of the laminate have a creped structure of a plurality of raised areas 14a, 16a separated by a plurality of non-raised areas 14b, 16b formed by the bonding sites of said bonding pattern 13. The creped structure of the first outer layer 14 is more distinct with a greater height h of said raised areas 14a as compared to the middle layer 16.

The second outer layer 15 of said laminate is substantially plane and uncreped and is preferably used as the internal side of the belt members intended to be facing the wearer, and should therefor be smooth and skin-friendly. The first outer layer 14 of the laminate is used as the external side of the belt members intended to act as receiving surface for said fastening means, and is especially used as a loop material for a complementary hook material of a hook-and-loop type fastening means 8 and 9. The creped structure of the layer 14 will provide an increased bulk and a three-dimensional structure suitable for engaging a hook material. The loop function for the creped material is thus improved as compared to a substantially plane and smooth nonwoven material. Examples of nonwoven materials suitable for the first outer layer 14 are spunbond, meltblown, carded bonded materials etc. The other outer layer 15, intended to form an inner layer of the belt facing the wearer, should be of a soft and skin friendly fibrous material. Examples of suitable materials are spunbond and meltblown materials, carded bonded materials etc. Examples of polymer materials used in the different fibrous materials may be any suited for this purpose, for example polypropylene, polyethylene, polyester and /or so called bicomponent fibers. The middle layer 16 is used as a support layer providing strength and stability to the laminate. The middle layer 16 should be of a relatively tear strong fibrous material, such as a spunbond or meltblown material comprising continuous filaments.

The laminate should have a tear strength of at least 22 N. This will make the belt members resist tearing as the belt is tightened around the waist of the wearer. Tests have proven that the tearing frequency at normal use for belts having a tear strength of 21 N and lower was unaccetably high. Preferably the tear strength should be at least 24 N, more preferably at least 25 N and most preferably at least 27N. For those belt having a tear strength of 29 N or higher there were no tearing at all.

The tear strength is measured by EDANA test method TEAR 70.3-96 with the modification that a conditioning time of 4h, a temperature of 23°C and a relative humidity of 50% R.H. is used.

A bonding area of more than 10% will result in an increased amount of tearing indications or notches and an increased risk for tearing of the belt members.

Preferably the bonding area should be no more than 8% and more preferably no more than 5%.

The bonding pattern comprises a plurality of bonding sites in the form of points, lines, spots or the like arranged in a pattern. The bonding area of a bonding pattern is defined as the amount of the pattern that consists of the bonding sites.

Another important factor for providing high tear strength is the bonding tightness, which is the number of bonding sites per area unit. It is preferred that the bonding pattern 13 has a bonding tightness of between 1 and 15 bonding sites per cm². Preferably it has a bonding tightness of between 1 and 10 bonding sites per cm². With a high bonding tightness more tearing indications or notches are formed, which will deteriorate the tearing strength.

Relatively large bonding sites, for example in the form of lines, provides a relatively large bonding area with a smaller number of bonding sites, as compared to a bonding pattern of small bonding sites, for example in the form of points, arranged with a higher bonding tightness. Thus both bonding area and bonding tightness are important.

One non-limiting example of a laminate according to the invention is a three-layered laminate:
Carded thermobonded material, basis weight 30 gsm, PP fibers of 2.2 dtex;
Spunbond layer, basis weight 40 gsm, PP fibers of 2.2 dtex;
Carded thermobonded material, basis weight 22 gsm, PP fibers of 2.2 dtex.

The spunbond layer is used as the middle layer, the carded material having the highest basis weight is creped and intended to be used as outside of the belt and is adapted to act as loop material for a hook-and-loop type fastener and the carded material having the lowest basis weight is used as inner skin-facing side of the belt. The middle spunbond layer is also creped, but with a less distinct creped structure as compared to the carded material intended to be used as outside of the belt.

The laminate is bonded by ultrasonic bonding with a bonding area of about 3% and a bonding tightness of about 7 bonding sites per cm². The tear strength is 55N.

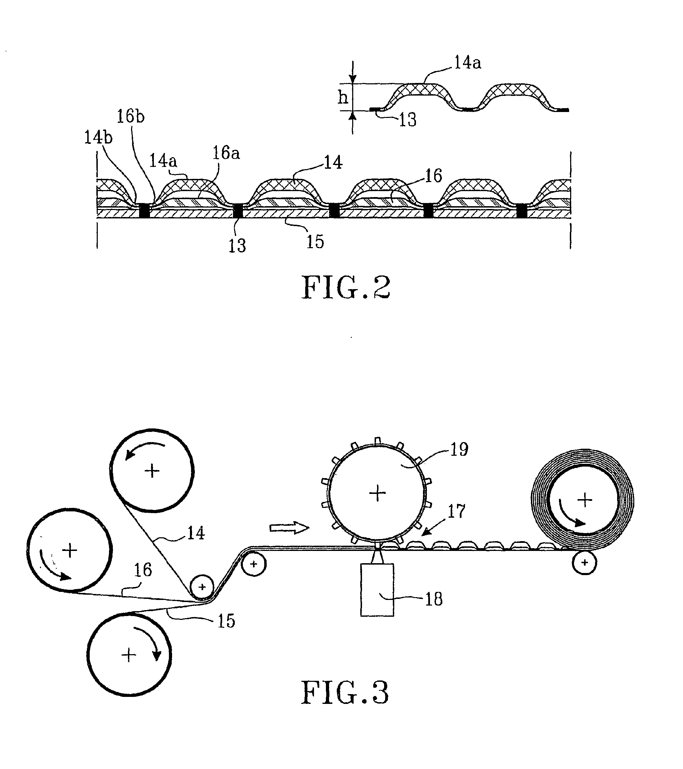

The method for manufacturing the laminate material according to the invention comprises binding together at least three layers 14, 15 and 16 of fibrous material in a bonding pattern 13 provided by ultrasonic, laser and/or heat in a bonding station 17 schematically illustrated in Fig. 3. The bonding station 17 in one embodiment of the invention comprises an ultrasonic horn 18 arranged opposite a patterning roll 19. In order to provide the creped structure the layers are fed with different web tensions and/or web speeds into the bonding station 17, at which the layer 14 exhibiting the lowest web tension and/or web speed is slowed down and becomes corrugated or creped at the feed end of the bonding station. The layer 15 exhibiting the highest web tension and/or web speed will remain substantially smooth, while the middle layer 16 exhibiting a web tension and/or web speed that is higher than that of the layer 14 but lower than that of the layer 16, will also become corrugated or creped but to a less extent than the layer 14. After bonding the laminate is fed from the bonding station with a web tension/web speed that is substantially equal to that of the lowest web tension/web speed, namely that of the layer 14.

According to one embodiment the second outer layer 15 has a web tension and/or web speed entering the bonding station that is between 15 and 50% and preferably between 18 and 33% higher than that of the first outer layer 14. According to a further embodiment the middle layer 16 has a web tension and/or web speed entering the bonding station that is between 5 and 40% and preferably between 9 and 18% higher than that of the first outer layer.

## Claims

1. Absorbent article such as a diaper and an incontinence guard comprising a liquid permeable topsheet (2), a liquid impermeable backsheet (3) and an absorbent body (4) enclosed therebetween, said article having a front portion (5), a rear portion (6) and a crotch portion (7) therebetween, and further is provided with a pair of belt members (10 a, b) attached to the rear portion (6), alternatively to the front portion, of the article and which are intended to be fastened together around the waist of the wearer by fastening means (11,12) and where said front portion (5), alternatively said rear portion, is provided with fastening means (8,9) intended to be fastened to the belt members (10 a, b), in such a way that the article will assume a pantlike shape, where the belt members (10 a,b) form a part of the waist portions of the pant,
**characterized in**
**that** the belt members (10 a, b) comprise a flexible laminate of at least three layers, a first outer layer (14), a middle layer (16) and a second outer layer (15) of fibrous material bonded together in a bonding pattern (13) provided by ultrasonic, laser and/or heat, said bonding pattern (13) having a bonding area of no more than 10% , said first outer layer (14) and said middle layer (16) of the laminate having a creped structure of a plurality of raised areas (14a;16a) separated by a plurality of non-raised areas formed by the bonding sites (13) of said bonding pattern, wherein the creped structure of the first outer layer (14) is more distinct with a greater height (h) of said raised areas (14a) as compared to the middle layer (16).

2. Absorbent article as claimed in claim 1,
**characterized in**
**that** the second outer layer (15) of said laminate is substantially smooth and uncreped.

3. Absorbent article as claimed in claim 1or 2,
**characterized in**
**that** the second outer layer (15) of the laminate is used as the internal side of the belt members (10a, b) intended to be facing the wearer, while the first outer layer (14) of the laminate is used as the external side of the belt members intended to act as receiving surface for said fastening means (8, 9).

4. Absorbent article as claimed in claim 3,
**characterized in**
**that** the first outer layer (14) is used as a loop material for a complementary hook material of a hook-and-loop type fastening means (8, 9).

5. Absorbent article as claimed in any of the preceding claims,
**characterized in**
**that** said bonding pattern (13) has a bonding area of no more than 8% and preferably no more than 5%.

6. Absorbent article as claimed in any of the preceding claims,
**characterized in**
**that** said bonding pattern (13) has a tightness of bonding sites of between 1 and 15 bonding sites per cm².

7. Absorbent article as claimed in claim 6,
**characterized in**
**that** said bonding pattern (13) has a tightness of bonding sites of between 1 and 10 bonding sites per cm².

8. Absorbent article as claimed in claim 7,
**characterized in**
**that** said middle layer (16) is a relatively tear strong fibrous material comprising continuous filaments, such as a spunbond and/or meltblown material.

9. Method of producing a flexible laminate for use as belt members (10a, b ) on an absorbent article such as a diaper and an incontinence guard of the kind stated in claim 1,
**characterized in**
binding together in a bonding station (17) at least three layers, a first outer layer (14), a middle layer (16) and a second outer layer (15) of fibrous material in a bonding pattern (13) provided by ultrasonic, laser and/or heat, said bonding pattern (13) having a bonding area of no more than 10% , said layers (14,15,16) of the laminate exhibiting different web tensions and/or web speeds when entering the bonding station (17), so that said first outer layer (14) exhibits the lowest web tension and/or lowest web speed, the second outer layer (15) exhibits the highest web tension and/or highest web speed and the middle layer (16) exhibits a web tension and/or web speed that is higher than that of the first outer layer (14) and lower than that of the second outer layer (15).

10. Method as claimed in claim 9,
**characterized in**
**that** the second outer layer (15) has a web tension and/or web speed during bonding that is between 15 and 50% higher than that of the first outer layer (14).

11. Method as claimed in claim 10,
**characterized in**
**that** the second outer layer (15) has a web tension and/or web speed during bonding that is between 18 and 33% higher than that of the first outer layer (14).

12. Method as claimed in any of claims 9-11,
**characterized in**
**that** the middle layer (16) has a web tension and/or web speed during bonding that is between 5 and 40% higher than that of the first outer layer (14).

13. Method as claimed in claim 12,
**characterized in**
**that** the middle layer (15) has a web tension and/or web speed during bonding that is between 9 and 18% higher than that of the first outer layer (14).

## Patentansprüche

1. Absorptionsartikel, wie z.B. eine Windel und ein Inkontinenzschutz, aufweisend eine flüssigkeitsdurchlässige Oberlage (2), eine flüssigkeitsundurchlässige Rücklage (3) und einen Absorptionskörper (4), der dazwischen eingeschlossen ist, wobei der Artikel einen Frontabschnitt (5), einen Rückabschnitt (6) und einen Schrittabschnitt (7) dazwischen aufweist, und der ferner mit einem Paar Gürtelelemente (10a, b) versehen ist, die an dem Rückabschnitt (6), alternativ an dem Frontabschnitt, des Artikels angebracht sind und die dazu vorgesehen sind, um die Taille des Trägers herum durch Befestigungsmittel (11, 12) aneinander befestigt zu werden, und wobei der Frontabschnitt (5), alternativ der Rückabschnitt, mit Befestigungsmitteln (8, 9) versehen ist, die dafür vorgesehen sind, an den Gürtelelementen (10a, b) auf eine solche Weise befestigt zu werden, dass der Artikel eine hosenähnliche Form annehmen wird, wobei die Gürtelelemente (10a, b) einen Teil der Taillenabschnitte der Hose bilden,
**dadurch gekennzeichnet,**
**dass** die Gürtelelemente (10a, b) ein flexibles Laminat von zumindest drei Schichten aufweisen, eine erste äußere Schicht (14), eine mittlere Schicht (16) und eine zweite äußere Schicht (15) aus Fasermaterial, das in einem Verbindungsmuster (13) zusammengebunden ist, das durch Ultraschall, Laser und/oder Wärme gebildet wird, wobei das Verbindungsmuster (13) eine Verbindungsfläche von nicht mehr als 10% aufweist, wobei die erste äußere Schicht (14) und die mittlere Schicht (16) des Laminats eine Kreppstruktur einer Mehrzahl erhöhter Flächen (14a; 16a) aufweisen, die durch eine Mehrzahl nicht erhöhter Flächen getrennt sind, die durch die Verbindungsstellen (13) des Verbindungsmusters gebildet sind, wobei die Kreppstruktur der ersten äußeren Schicht (14) im Vergleich mit der mittleren Schicht (16) mit einer größeren Höhe (h) der erhöhten Bereiche (14a) stärker ausgeprägt ist.

2. Absorptionsartikel nach Anspruch 1,
**dadurch gekennzeichnet,**
**dass** die zweite äußere Schicht (15) des Laminats im Wesentlichen glatt und nicht gekreppt ist.

3. Absorptionsartikel nach Anspruch 1 oder 2,
**dadurch gekennzeichnet,**
**dass** die zweite äußere Schicht (15) des Laminats als die Innenseite der Gürtelelemente (10a, b) verwendet wird, die dafür vorgesehen ist, dem Benutzer zugewandt zu sein, während die erste äußere Schicht (14) des Laminats als die Außenseite der Gürtelelemente verwendet wird, die dazu vorgesehen ist, als Aufnahmeoberfläche für die Befestigungsmittel (8, 9) zu wirken.

4. Absorptionsartikel nach Anspruch 3,
**dadurch gekennzeichnet,**
**dass** die erste äußere Schicht (14) als ein Schlaufenmaterial für ein komplementäres Hakenmaterial eines Klettbefestigungsmittels (8, 9) verwendet wird.

5. Absorptionsartikel nach einem der vorhergehenden Ansprüche,
**dadurch gekennzeichnet,**
**dass** das Verbindungsmuster (13) eine Verbindungsfläche von nicht mehr als 8% und bevorzugt nicht mehr als 5% aufweist.

6. Absorptionsartikel nach einem der vorhergehenden Ansprüche,
**dadurch gekennzeichnet,**
**dass** das Verbindungsmuster (13) eine Dichte von Verbindungsstellen von zwischen 1 und 15 Verbindungsstellen pro cm² hat.

7. Absorptionsartikel nach Anspruch 6,
**dadurch gekennzeichnet,**
**dass** das Verbindungsmuster (13) eine Dichte von Verbindungsstellen von zwischen 1 und 10 Verbindungsstellen pro cm² hat.

8. Absorptionsartikel nach Anspruch 7,
**dadurch gekennzeichnet,**
**dass** die mittlere Schicht (16) ein relativ reißfestes Fasermaterial ist, umfassend durchgehende Filamente, wie z.B. ein Spundbond oder Meltblown Material.

9. Verfahren zum Herstellen eines flexiblen Laminats zur Verwendung als Gürtelelemente (10a, b), an einem Absorptionsartikel, wie z.B. einer Windel oder einem Inkontinenzschutz der Art, die in Anspruch 1 definiert wird,
**gekennzeichnet durch**
miteinander Verbinden zumindest dreier Schichten in einer Verbindungsstation (17), einer ersten äußeren Schicht (14), einer mittleren Schicht (16) und einer zweiten äußeren Schicht (15) aus Fasermaterial, in einem Verbindungsmuster (13), das **durch** Ultraschall, Laser und/oder Wärme gebildet wird, wobei das Verbindungsmuster (13) eine Verbindungsfläche von nicht mehr als 10% aufweist, wobei die Schichten (14, 15, 16) des Laminats verschiedene Bahnspannungen und/oder Bahngeschwindigkeiten aufweisen, wenn sie in die Verbindungsstation (17) eintreten, so dass die erste äußere Schicht (14) die niedrigste Bahnspannung und/oder niedrigste Bahngeschwindigkeit aufzeigt, die zweite äußere Schicht (15) die höchste Bahnspannung und/oder höchste Bahngeschwindigkeit aufzeigt und die mittlere Schicht (16) eine Bahnspannung und/oder Bahngeschwindigkeit aufzeigt, die höher als die der ersten äußeren Schicht (14) und niedriger als die der zweiten äußeren Schicht (15) ist.

10. Verfahren nach Anspruch 9,
**dadurch gekennzeichnet,**
**dass** die zweite äußere Schicht (15) eine Bahnspannung und/oder Bahngeschwindigkeit während des Verbindens aufweist, die zwischen 15 und 50% höher als die der ersten äußeren Schicht (15) ist.

11. Verfahren nach Anspruch 10,
**dadurch gekennzeichnet,**
**dass** die zweite äußere Schicht (15) eine Bahnspannung und/oder Bahngeschwindigkeit während des Verbindens aufweist, die zwischen 18 und 33% höher als die der ersten äußeren Schicht (14) ist.

12. Verfahren nach einem der Ansprüche 9 bis 11,
**dadurch gekennzeichnet,**
**dass** die mittlere Schicht (16) eine Bahnspannung und/oder Bahngeschwindigkeit während des Verbindens hat, die zwischen 5 und 40% höher als die der ersten äußeren Schicht (14) ist.

13. Verfahren nach Anspruch 12,
**dadurch gekennzeichnet,**
**dass** die mittlere Schicht (15) eine Bahnspannung und/oder Bahngeschwindigkeit während des Verbindens hat, die zwischen 9 und 18% höher als die der ersten äußeren Schicht (14) ist.

## Revendications

1. Article absorbant comme une couche-culotte ou une protection contre l'incontinence comprenant une feuille supérieure (2) perméable aux liquides, une couche arrière (3) imperméable aux liquides et un corps absorbant (4) enfermé entre elles, ledit article ayant une partie avant (5), une partie arrière (6) et une partie d'entrejambe (7) entre elles, et est en outre pourvu d'une paire d'éléments de ceinture (10a, b) fixés à la partie arrière (6), respectivement à la partie avant, de l'article et qui sont destinés à être attachés l'un à l'autre autour de la taille du porteur par un moyen de fixation (11, 12) et où ladite partie avant (5), respectivement ladite partie arrière, est pourvue d'un moyen de fixation (8, 9) destiné à être attaché aux éléments de ceinture (10a, b), de manière telle que l'article prend une forme de culotte, où les éléments de ceinture (10a, b) forment une partie de la partie taille de la culotte,
**caractérisé en ce que** les éléments de ceinture (10a, b) comprennent une structure stratifiée flexible comportant au moins trois couches, une première couche extérieure (14), une couche médiane (16) et une deuxième couche extérieure (15) en matière fibreuse collées les unes aux autres selon un motif de liaison (13) réalisé par ultrasons, par laser et/ou par chaleur, ledit motif de liaison (13) ayant une surface de liaison ne dépassant pas 10 %, ladite première couche extérieure (14) et ladite couche médiane (16) de la structure stratifiée présentant une structure crêpée constituée d'une pluralité de zones en relief (14a ; 16a) séparées par une pluralité de zones non en relief formées par les sites de liaison (13) dudit motif de liaison, dans lequel la structure crêpée de la première couche extérieure (14) est plus distincte avec une plus grande hauteur (h) desdites zones en relief (14a) relativement à la couche médiane (16).

2. Article absorbant selon la revendication 1, **caractérisé en ce que** la deuxième couche extérieure (15) de ladite structure stratifiée est substantiellement lisse et non crêpée.

3. Article absorbant selon la revendication 1 ou 2, **caractérisé en ce que** la deuxième couche extérieure (15) de la structure stratifiée est employée comme face interne des éléments de ceinture (10a, b) destinée à être tournée vers le porteur, tandis que la première couche extérieure (14) de la structure stratifiée est employée comme face externe des éléments de ceinture destinée à servir de surface réceptrice pour ledit moyen de fixation (8, 9).

4. Article absorbant selon la revendication 3, **caractérisé en ce que** la première couche extérieure (14) sert de matériau à boucles pour un matériau complémentaire à crochets d'un moyen de fixation du type à crochets et à boucles (8, 9).

5. Article absorbant selon l'une quelconque des revendications précédentes, **caractérisé en ce que** ledit motif de liaison (13) a une surface de liaison ne dépassant pas 8 % et de préférence ne dépassant pas 5 %.

6. Article absorbant selon l'une quelconque des revendications précédentes, **caractérisé en ce que** ledit motif de liaison (13) a une densité de sites de liaison comprise entre 1 et 15 sites de liaison par cm².

7. Article absorbant selon la revendication 6, **caractérisé en ce que** ledit motif de liaison (13) a une densité de sites de liaison comprise entre 1 et 10 sites de liaison par cm².

8. Article absorbant selon la revendication 7, **caractérisé en ce que** ladite couche médiane (16) est faite d'une matière fibreuse relativement résistante à la déchirure, comprenant des filaments continus, comme une matière filée-liée et/ou fondue-soufflée.

9. Procédé de fabrication d'une structure stratifiée flexible destinée à être utilisée comme éléments de ceinture (10a, b) sur un article absorbant comme une couche-culotte ou une protection contre l'incontinence du type décrit dans la revendication 1, **caractérisé par** le fait de lier entre elles dans une station de collage (17) au moins trois couches, une première couche extérieure (14), une couche médiane (16) et une deuxième couche extérieure (15) en matière fibreuse selon un motif de liaison (13) réalisé par ultrasons, par laser et/ou par chaleur, ledit motif de liaison (13) ayant une surface de liaison ne dépassant pas 10 %, lesdites couches (14, 15, 16) de la structure stratifiée présentant des tensions de bande et/ou des vitesses de bande différentes lors de leur entrée dans la station de collage (17), de sorte que ladite première couche extérieure (14) présente la plus basse tension de bande et/ou la plus basse vitesse de bande, la deuxième couche extérieure (15) présente la plus haute tension de bande et/ou la plus haute vitesse de bande et la couche médiane (16) présente une tension de bande et/ou une vitesse de bande qui est supérieure à celle de la première couche extérieure (14) et inférieure à celle de la deuxième couche extérieure (15).

10. Procédé selon la revendication 9, **caractérisé en ce que** la deuxième couche extérieure (15) présente une tension de bande et/ou une vitesse de bande pendant le collage qui est de 15 à 50 % supérieure à celle de la première couche extérieure (14).

11. Procédé selon la revendication 10, **caractérisé en ce que** la deuxième couche extérieure (15) présente une tension de bande et/ou une vitesse de bande pendant le collage qui est de 18 à 33 % supérieure à celle de la première couche extérieure (14).

12. Procédé selon l'une quelconque des revendications 9 à 11, **caractérisé en ce que** la couche médiane (16) présente une tension de bande et/ou une vitesse de bande pendant le collage qui est de 5 à 40 % supérieure à celle de la première couche extérieure (14).

13. Procédé selon la revendication 12, **caractérisé en ce que** la couche médiane (15) présente une tension de bande et/ou une vitesse de bande pendant le collage qui est de 9 à 18 % supérieure à celle de la première couche extérieure (14).
